# DEMANDE DE BREVET EUROPEEN

(11) **EP 0 741 999 A1**
(43) Date de publication de la demande: **13.11.1996**
(21) Numéro de dépôt: 96490022.9
(22) Date de dépôt: 03.05.1996
(51) Int. Cl.: A61F 13/15

(54) **Dispositif pour la fabrication de couches-culottes à ceinture élastique et couches-culottes obtenues**

(30) Priorité: 11.05.1995 FR 9505585
(71) Demandeur: INBRAND FRANCE Société Anonyme, 59290 Wasquehal (FR); CELLULOSE CONVERTING EQUIPMENT Srl, 65100 Pescara (IT)
(72) Inventeur: Lahousse, Thierry, 59223 Roncq (FR)
(74) Mandataire: Duthoit, Michel

(57) **Abrégé**

L'invention concerne un nouveau dispositif pour la fabrication de couches-culottes à ceinture élastique utilisables dans le domaine de l'hygiène infantile et/ou médicale, caractérisé en ce qu'il comporte des premiers moyens d'étirement et de maintien à l'état étiré desdits pavés (15) de matière formant la ceinture élastique (14) avant et pendant le collage sur la matière formant le voile interne (13) et des seconds moyens de maintien desdits pavés (15) de matière formant la ceinture élastique (14) collée sur ladite matière formant le voile interne (13) pratiquement jusqu'au collage desdits pavés (15) collés de ladite matière formant le voile interne (13) sur ladite matière formant la feuille extérieure (18).

## Description

L'invention concerne un nouveau dispositif pour la fabrication de couches-culottes à ceinture élastique.

Elle se rapporte également à une nouvelle couche-culotte à ceinture élastique.

De façon générale, les couches-culottes utilisables pour l'hygiène infantile et/ou médicale sont formées d'une partie avant et d'une partie arrière réunies par une partie centrale formant l'entrejambe et comprennent :
une feuille extérieure imperméable;
un voile interne perméable de confort;
une couche absorbante, éventuellement munie d'un tampon central, disposée entre ladite feuille extérieure et ledit voile interne, ainsi que des moyens de fermeture de la couche au niveau de la ceinture.

L'un des perfectionnements apportés aux couches-culottes depuis quelques années consiste à prévoir une bande élastique en mousse, disposée entre la feuille extérieure et le voile interne, qui permet de froncer légèrement la couche au niveau de la ceinture.

Cette bande élastique a principalement pour but d'adapter la grandeur de la ceinture en fonction des variations de morphologie d'un même utilisateur suivant sa position ou d'utilisateurs différents.

On a remarqué par exemple, que lorsqu'un utilisateur passe d'une position debout à une position couchée, sa taille n'a pas exactement la même morphologie. De même, entre deux bébés de même âge, les morphologies ne sont généralement pas identiques.

La présence de la bande élastique permet donc de pallier à ces différences de morphologie, sans être obligé de changer fréquemment l'utilisateur ou de trouver une couche-culotte s'adaptant parfaitement à un utilisateur donné.

La bande élastique renforce également l'étanchéité de la couche-culotte, en maintenant de façon plus serrée la couche-culotte contre la taille de l'utilisateur.

La fabrication des couches-culottes à ceinture élastique est classiquement réalisée de la façon suivante.

Les éléments constitutifs de la couche-culotte, à savoir la feuille extérieure, le voile interne et la bande élastique sont généralement disponibles sous forme de rouleaux qui peuvent être déroulés en bandes continues. Le processus conventionnel de fabrication d'une couche-culotte à ceinture élastique est alors le suivant :

La bande de matière formant la bande élastique est placée sur différents rouleaux d'amenée, coupée aux dimensions voulues de façon à former des "pavés". Un mécanisme d'étirement des pavés de bande élastique formés est ensuite prévu pour coller les pavés de bande élastique sur le voile interne, dans un état étiré.

Après que le pavé de bande élastique soit fixé sur le voile interne, la bande élastique revient dans sa forme originale, en raison de son élasticité naturelle, conduisant à l'apparition de fronces au niveau de la ceinture.

Différentes méthodes pour provoquer l'étirement des pavés de bande élastique ont été proposées.

Une première méthode consiste à enfoncer, au moins partiellement, des épingles aux extrémités de la bande pour la maintenir et l'étirer de cette façon, au fur et à mesure de son déplacement. Ce système souffre cependant de l'inconvénient majeur d'abîmer la bande élastique en y provoquant des trous fins qui peuvent fragiliser la ceinture élastique obtenue.

De plus, le risque existe que certaines épingles cassent pendant l'étirement de la bande élastique et que des fragments restent coincés dans la bande élastique.

On conçoit alors que de tels fragments, suivant la position dans laquelle ils restent dans la couche-culotte, peuvent blesser ou à tout le moins gêner l'utilisateur du produit.

Une seconde méthode d'étirement de la bande élastique a été proposée dans laquelle deux poulies d'écartement assurent l'étirement de la bande élastique.

Dans ce système, l'axe de rotation des poulies forme un angle ouvert vers l'extérieur avec l'axe de déplacement de la bande élastique pendant le processus de fabrication. Les deux poulies sont placées de chaque côté de la bande élastique (ou du pavé de bande élastique) et comportent chacune une courroie torique permettant de coincer chaque extrémité de la bande élastique entre la courroie et la surface de la poulie correspondante.

Ainsi, au fur et à mesure que les poulies tournent, elles entraînent le déplacement de la bande élastique maintenue par ses bords et l'étirent progressivement.

Le dispositif est réglé de façon que les courroies maintiennent la bande à l'état étiré jusqu'au moment de son transfert sur le voile interne préalablement encollé. De cette façon, la bande étirée est collée sur le voile interne en gardant sa forme étirée après avoir été libérée des courroies.

Ce second système souffre cependant de divers inconvénients.

L'un des principaux inconvénients réside dans le fait que lorsque la bande étirée est placée sur le voile interne, son maintien dans une position étirée d'une part et son positionnement par rapport audit voile interne d'autre part ne sont plus assurés que par la colle déposée sur le voile interne. Il arrive alors qu'un certain nombre de bandes élastiques, principalement en raison du temps nécessaire au durcissement de la colle, reprennent, au moins en partie, leur état non étiré ou ne gardent pas leur position initiale sur le voile interne. On aboutit par conséquent à un taux de déchet non négligeable parmi les couches-culottes fabriquées.

Par ailleurs, ce système qui comporte déjà les inconvénients décrits dans ce qui précède lorsqu'il est utilisé avec des bandes élastiques en mousse, se révèle n'être plus du tout adapté lorsque des bandes élastiques en matière plastique beaucoup plus fine que les mousses sont utilisées.

L'invention a pour but de remédier en grande partie à ces inconvénients, en proposant un nouveau dispositif pour la fabrication de couches-culottes à ceinture élastique, permettant de fabriquer aussi bien des couches-culottes dans lesquelles la bande élastique est en mousse que des couches-culottes dans lesquelles la bande élastique est en matière plastique fine, et ce avec des taux de pertes minimes.

L'invention a également pour but de proposer une nouvelle couche-culotte à ceinture élastique pouvant être préparée par la mise en oeuvre du dispositif selon l'invention.

Le nouveau dispositif selon l'invention, pour la fabrication de couches-culottes à ceinture élastique utilisables dans le domaine de l'hygiène infantile et/ou médicale, comprenant des moyens de défilement sous forme de bandes continues de la matière formant la feuille extérieure, de la matière formant la ceinture élastique, de la matière formant le voile interne et de la matière formant la couche absorbante; des moyens de découpe de ladite matière formant la ceinture élastique, de façon à former des pavés de ladite matière formant la ceinture élastique; des moyens d'encollage de la matière formant le voile interne et de la matière formant la feuille extérieure et des moyens de transfert dudit pavé de matière formant la ceinture élastique collé sur ladite matière formant le voile interne sur ladite matière formant feuille extérieure, se caractérise en ce qu'il comprend des moyens de maintien de la matière formant la bande élastique, lesdits moyens agissant depuis le début de l'étirement de ladite matière formant la bande élastique et pratiquement jusqu'au collage de la matière formant la feuille extérieure.

Selon l'invention, les moyens de maintien comprennent des premiers moyens d'étirement et de maintien à l'état étiré desdits pavés de matière formant la ceinture élastique avant et pendant le collage de ceux-ci sur la matière formant le voile interne et des seconds moyens de maintien desdits pavés de matière formant la ceinture élastique collés sur ladite matière formant le voile interne pratiquement jusqu'au collage de ladite matière formant le voile interne sur ladite matière formant la feuille extérieure.

Selon l'invention, les pavés de matière formant la bande élastique sont en premier lieu étirés par des premiers moyens d'étirement et/ou de maintien.

Selon une forme de réalisation préférée de la présente invention, les premiers moyens d'étirement et de maintien comprennent au moins deux poulies d'écartement. Chaque poulie comporte une gorge interne et une gorge externe aménagées sur sa surface extérieure, la gorge externe recevant une courroie torique supérieure et la gorge interne recevant une courroie torique inférieure. Lorsque le pavé de matière formant la bande élastique arrive au niveau des poulies, les bords de celui-ci sont coincés entre la courroie supérieure et la gorge externe et il est maintenu dans cette position grâce à la tension de la courroie supérieure. L'angle ouvert formé entre l'axe de rotation des poulies et l'axe de déplacement de la matière formant le pavé de bande élastique permet alors d'étirer progressivement le pavé pendant son déplacement, tout en le maintenant par coincement de ses bords.

Selon l'invention, les pavés de matière formant la bande élastique, après leur collage sur la matière formant le voile interne, sont encore maintenus par des seconds moyens de maintien. Avantageusement, ces seconds moyens comprennent au moins deux courroies toriques inférieures passant par les gorges internes des poulies d'écartement.

Avantageusement, le trajet des courroies supérieure et inférieure passe respectivement par des roues supérieure et inférieure, au moins partiellement.

Selon l'invention, le déplacement de la matière formant la bande élastique passe donc par un rouleau supérieur, par les poulies d'écartement puis par un rouleau inférieur.

Lors de ces transferts, l'état physique de la matière formant la bande élastique varie. En effet, sur le rouleau supérieur, la matière formant la bande élastique est coupée en un pavé qui est maintenu à l'état non étiré sur la surface du rouleau supérieur par tout moyen connu, par exemple grâce à un système d'aspiration d'air.

Sur les poulies d'écartement, le pavé est maintenu par coincement de ses bords par les courroies supérieures et est progressivement étiré.

Enfin, sur le rouleau inférieur, le pavé est collé sur la matière formant le voile interne, en position étirée et est maintenu dans cette position par les courroies inférieures.

Le positionnement des rouleaux et des poulies les uns par rapport aux autres, ainsi que la longueur du trajet des courroies, ne sont pas critiques en eux-mêmes, et peuvent varier en fonction du type d'installation et/ou des matériaux utilisés pour la fabrication des couches-culottes.

L'homme du métier aura toute possibilité, par des essais de mises au point, d'adapter ces éléments de façon à assurer un rendement maximal du dispositif de la présente invention, en respectant cependant les conditions décrites ci-après.

En particulier, les courroies supérieures ne font pas nécessairement tout le tour de la circonférence du rouleau supérieur et des poulies d'écartement.

De même, les courroies inférieures ne font pas nécessairement tout le tour de la circonférence des poulies d'écartement et du rouleau inférieur.

Il doit être compris que chaque courroie définit en effet un point d'entrée à l'endroit où elle vient en contact avec un rouleau ou une poulie et définit un point de sortie à l'endroit où elle n'est plus en contact avec ledit rouleau ou ladite poulie.

Ainsi, la zone de transfert d'un pavé de bande élastique du rouleau supérieur sur les poulies d'écartement coïncide avec le point d'entrée des courroies supérieures, de façon que le pavé, maintenu par exemple par aspiration sur le rouleau supérieur, devienne maintenu par les courroies supérieures dès qu'il passe sur les poulies d'écartement.

De même, dans la zone de transfert du pavé étiré des poulies d'écartement sur le rouleau inférieur, le point de sortie des courroies supérieures coïncide avec le point d'entrée des courroies inférieures, de façon que le pavé étiré ne se rétracte pas ou ne bouge pas lorsqu'il passe, en étant collé sur la matière formant le voilé interne, sur le rouleau inférieur.

Avantageusement, on peut prévoir dans cette dernière zone de transfert, que le point d'entrée des courroies inférieures soit situé en amont du point de sortie des courroies supérieures, par rapport au sens de déplacement du pavé.

Ainsi, le maintien du pavé par les courroies inférieures commence avant même que ne finisse le maintien du pavé par les courroies supérieures.

Le dispositif selon l'invention comprend de plus des moyens d'application d'une force de plaquage du pavé de bande élastique collé sur le voile interne et de la matière formant feuille externe pratiquement immédiatement après collage sur la matière formant voile interne. Ces moyens de plaquage comprennent des rouleaux venant en appui, au moins temporairement, contre le rouleau recevant les pièces de la couche-culotte venant d'être assemblées par collage.

De cette façon, la liaison et le maintien dans le positionnement original des pièces de la couche-culotte sont encore renforcés, les rouleaux lors de leur passage contre lesdites pièces réduisant les risques de mouvement des pièces les unes par rapport aux autres et améliorant la liaison collante entre les pièces.

L'invention concerne également une nouvelle couche-culotte à ceinture élastique.

La nouvelle couche-culotte à ceinture élastique utilisable pour l'hygiène infantile et/ou médicale, formée d'une partie avant et d'une partie arrière réunies par une partie centrale formant l'entrejambe, ladite couche comprenant :
une feuille extérieure imperméable;
un voile interne perméable de confort;
une couche absorbante disposée entre ladite feuille extérieure et ledit voile interne, ladite couche absorbante étant éventuellement munie d'un tampon central;
ladite couche-culotte présentant en outre des moyens d'élastification de la ceinture ainsi que des moyens de fermeture de la couche au niveau de la ceinture, se caractérise en ce que lesdits moyens d'élastification comprennent une bande élastique d'une épaisseur comprise entre environ 50 µm et environ 90 µm, de préférence entre environ 50 µm et environ 70 µm.

Avantageusement, la feuille extérieure est réalisée en polyéthylène, d'une épaisseur comprise entre environ 20 et environ 30 µm, de préférence de 25 µm et possède une densité basse, variant entre environ 20 g/m² et environ 30 g/m², de préférence de 24 g/m².

La couche absorbante et le tampon central sont généralement réalisés en cellulose, en fibres synthétiques, etc.

Le voile interne est réalisé de préférence en un matériau non tissé, par exemple en polypropylène hydrophile, en matériaux sous forme de fils tels que spun, spun cardé, etc. Le voile interne possède une densité variant entre environ 10 g/m² et environ 30 g/m², de préférence de 20 g/m².

De préférence, la bande élastique possède un étirement maximal par rapport à sa limite d'élasticité d'environ 80 %, par rapport à sa longueur initiale.

De préférence, la bande élastique est réalisée à partir de matière plastique, notamment à partir de polypropylène ou ses dérivés, à partir de latex ou ses dérivés, à partir de caoutchouc naturel ou synthétique ou ses dérivés, ainsi que les mélanges de ces produits.

On citera, à titre d'exemple non limitatif de matière plastique utilisable, le produit FLUTED ELASTIC® commercialisé par la société Minnesota Mining Manufacturing (3M), France.

La nouvelle couche-culotte à ceinture élastique selon l'invention est fabriquée avantageusement avec le dispositif de l'invention décrit dans ce qui précède. En effet, les dispositifs de l'art antérieur évoqués dans la partie introductrice de la présente demande de brevet, s'ils permettent la fabrication, avec les inconvénients exposés plus haut, de couches-culottes comportant une ceinture élastique en mousse, ne conviennent plus du tout lorsqu'il s'agit de fabriquer une couche-culotte comportant une ceinture élastique réalisée en une matière beaucoup plus fine, telle que celles qui viennent d'être décrites.

Il va de soi cependant que la couche-culotte selon l'invention, nouvelle en elle-même, ne saurait être limitée par son procédé de fabrication utilisant le dispositif de la présente invention.

La couche-culotte selon l'invention permet donc d'obtenir un produit comportant une ceinture élastique ne formant pas de "sur-épaisseur" au niveau de la bande élastique, du fait de la faible épaisseur de celle-ci. Elle permet par conséquent d'offrir un produit d'un aspect beaucoup plus homogène et d'un confort amélioré, les risques de démangeaisons ou d'irritations dus à la présence d'une zone d'épaisseur plus importante que le reste de la ceinture étant éliminés pratiquement complètement.

Des avantages et caractéristiques supplémentaires de l'invention apparaîtront encore à la lumière de la description plus détaillée qui suit d'exemples de réalisation de la présente invention, donnés uniquement à titre illustratif et non limitatif, ainsi qu'aux figures qui s'y rapportent et dans lesquelles :
- la figure 1 représente une vue schématique de dessus d'un mode de réalisation du dispositif de la présente invention;
- la figure 2 représente une vue schématique de face du mode de réalisation de la figure 1;
- la figure 3 représente une vue schématique en perspective d'une couche-culotte selon la présente invention; et
- la figure 4 représente une vue schématique de dessus et à l'état étalé de la couche-culotte de la figure 3.

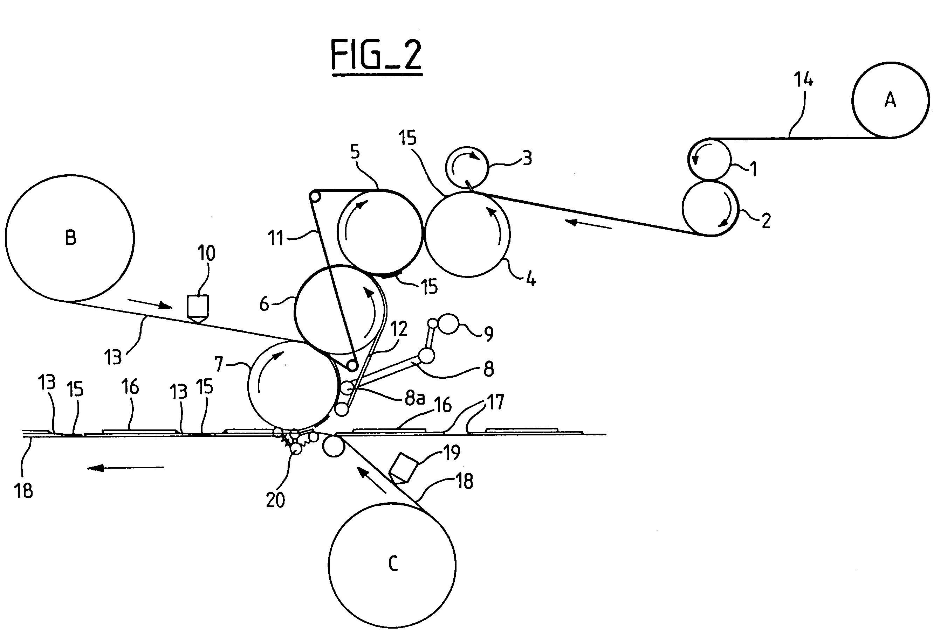

Sur la figure 2, les flèches indiquent le sens de rotation des rouleaux et le sens de déplacement des bandes continues de matières premières.

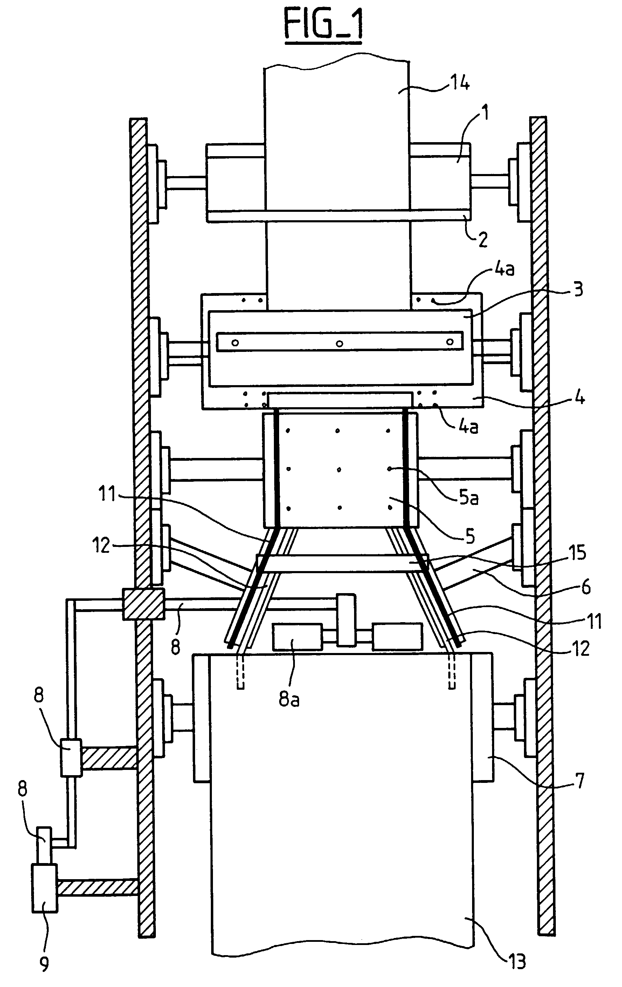

En se référant maintenant aux figures 1 et 2, le dispositif selon l'invention comprend un dévidoir A sur lequel est enroulée la matière formant la bande élastique 14. La bande élastique 14 passe entre un rouleau presseur 1 et un rouleau d'entraînement 2 motorisé, de façon connue en soi. Le rouleau presseur 1 a essentiellement un rôle d'amélioration de l'entraînement de la matière formant la bande élastique 14.

La matière formant la bande élastique 14 arrive ensuite entre un rouleau-enclume 4 et un rouleau porte-couteau 3. A ce stade du processus de fabrication, la matière formant la bande élastique 14 est coupée en pavés 15 de largeur déterminée, en fonction de la taille du produit final.

Généralement, les couches-culottes étant fabriquées en continu, la largeur du pavé 15 sera le double de la largeur désirée pour une couche-culotte finale, le pavé 15 étant ultérieurement coupé de façon à ce que chaque partie de pavé coupée forme la ceinture élastique de deux couches-culottes adjacentes.

A chaque tour de la roue à vide 4, le couteau 3 réalise deux pavés 15.

Le rouleau à vide 4 comporte à sa surface des orifices 4a en communication avec un système conventionnel d'aspiration d'air. De cette façon, la bande élastique 14 et les pavés 15 après passage du couteau porté par le rouleau 3 sont maintenus correctement en place sur la surface du rouleau 4.

La roue à vide 4 tourne de plus à une vitesse plus importante que le rouleau 2, permettant ainsi une tension constante de la bande 14 nécessaire pour que le rouleau porte-couteau 3 opère une coupe satisfaisante.

Le pavé 15 arrive ensuite à une première zone de transfert, où il passe du rouleau 4 à un rouleau supérieur à vide 5. L'aspiration prévue sur le rouleau 4 est réglée de façon à s'arrêter lorsqu'un pavé 15 arrive à la surface de contact entre les rouleaux 4 et 5. Le pavé 15 n'étant plus maintenu par aspiration sur le rouleau 4, il est "attiré" par le rouleau supérieur 5 qui dispose également d'un système d'aspiration en communication avec les orifices 5a. Le rouleau supérieur 5 est muni de deux courroies toriques supérieures 11 passant autour d'une partie de sa circonférence, et les pavés 15 sont maintenus sur le rouleau 5 par aspiration, au-dessus des courroies supérieures 11.

Le pavé 15 arrive ensuite à une seconde zone de transfert où il passe du rouleau 5 sur les poulies d'écartement 6. Les poulies d'entraînement comportent deux gorges externes (non représentées sur les figures 1 et 2) permettant le passage des courroies toriques supérieures 11.

A cette position, le retournement des courroies supérieures 11 entraîne que les bords du pavé 15 passent en-dessous des courroies supérieures 11 et au-dessus des gorges dans lesquelles glissent les courroies supérieures 11. Compte-tenu de la tension des courroies supérieures 11, le pavé 15 est "pincé" par ses bords et étiré au fur et à mesure de son déplacement, en raison de l'angle ouvert que forme l'axe de rotation des poulies 6 avec l'axe de déplacement du pavé 15, ainsi que cela apparaît sur la figure 1.

Les poulies d'écartement 6 comportent également deux gorges internes (non représentées sur les figures 1 et 2) dans lesquelles glissent les courroies inférieures 12. A ce stade du processus de fabrication, le pavé 15 est positionné au-dessus des courroies inférieures 12.

Le pavé 15 étiré arrive ensuite à une troisième zone de transfert où il passe des poulies d'écartement 6 à un rouleau inférieur 7. Le rouleau inférieur 7 fait défiler la matière formant le voile interne 13 à partir d'un dévidoir B. Avant que la matière formant le voile interne 13 n'arrive sur le rouleau 7, une station de collage 10 dépose sur la surface du voile interne 13 une épaisseur appropriée de colle.

Au moment où le pavé 15 passe des poulies d'écartement 6 sur le rouleau 7, le retournement des courroies inférieures 12 entraîne que le pavé 15 est pincé par les courroies inférieures 12 passant sur le rouleau 7 puis est dégagé des courroies supérieures 11. Le pavé 15 se trouve alors collé sur le voile interne 13 porté par le rouleau 7 et maintenu en place et à l'état étiré par les courroies inférieures 12.

Immédiatement après son collage sur le voile interne 13, le collage et le bon positionnement du pavé 15 sont renforcés par passage entre le rouleau 7 et un rouleau 8a relié par un levier 8 à une came 9. Avantageusement, le rouleau 8a vient frapper le rouleau inférieur 7 à chaque passage d'un pavé 15 collé sur le voile interne 13.

Un dévidoir C fait défiler la feuille externe 18 et dépose à sa surface une épaisseur de colle grâce à la rampe de collage 19. La feuille externe 18 encollée reçoit la couche absorbante 17 munie d'un tampon central 16, la couche absorbante 17 et le tampon central 16 provenant d'un autre dévidoir (non représenté sur les figures 1 et 2).

A la partie inférieure du rouleau inférieur 7, le voile interne 13 encollé et muni du pavé 15 vient en contact avec la feuille externe 18 munie de la couche absorbante 17 et du tampon central 16 et sont collés l'un sur l'autre, afin de former le produit final. Un rouleau 20 appuyant sur la partie inférieure du rouleau inférieur 7 par des ressorts exerce une force de pression sur la couche-culotte formée afin de renforcer le collage des différentes pièces les unes sur les autres.

La bande continue obtenue de couches-culottes est ensuite coupée au niveau du milieu du pavé de bande élastique 15 et les éléments adhésifs de fixation de la couche-culotte sont ensuite placés.

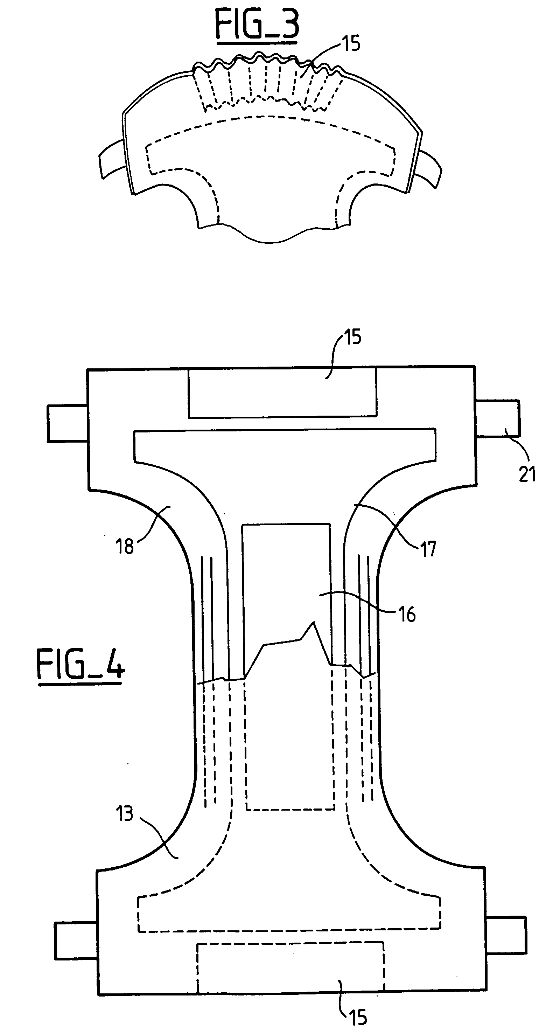

Les figures 3 et 4 représentent la couche-culotte obtenue par le dispositif selon l'invention.

En se référant maintenant à ces figures, la couche-culotte est formée d'une feuille extérieure 18 imperméable. La feuille extérieure 18 est réalisée en polyéthylène, d'une épaisseur de 25 µm et possède une densité basse de 24 g/m².

A l'intérieur de la feuille extérieure 18 est disposée une couche absorbante 17 munie d'un tampon central 16. La couche absorbante 17 et le tampon central 16 sont réalisés en cellulose.

La couche absorbante, le tampon central et la feuille extérieure sont recouverts du côté de la couche-culotte en contact avec la peau, d'un voile interne perméable de confort 13. Le voile interne 13 est réalisé en un matériau non tissé, en polypropylène hydrophile. Le voile interne possède une densité de 20 g/m².

Au niveau de la ceinture de la couche-culotte, un pavé élastique 15 est placé entre le voile interne 13 et la feuille extérieure 18 de façon à former des fronces pour ajuster correctement la couche-culotte à l'utilisateur.

Selon une forme de réalisation, le pavé élastique est réalisé en mousse, notamment en mousse de polyuréthanne d'une épaisseur de 2 mm, tel que par exemple le produit mousse commercialisé par la société Recticel, France.

Selon une autre forme de réalisation, le pavé élastique consiste en une bande de faible épaisseur en matière élastique, telle que celle commercialisée sous la dénomination FLUTED ELASTIC®.

Des éléments adhésifs 21 permettent enfin de fixer la couche-culotte à la taille de l'utilisateur.

Il va de soi que l'invention telle que décrite dans ce qui précède ne se limite nullement aux formes de réalisation qui viennent d'être décrites et qui ne sont données qu'à titre purement illustratif, mais en embrasse au contraire toute les variantes.

L'homme du métier aura toute possibilité de changer certains des éléments de la présente invention, dans le cadre de mises au point habituelles destinées à améliorer et optimiser la mise en oeuvre et les produits objets de la présente invention, sans pour autant sortir du cadre de ses éléments caractéristiques, tels que définis dans les revendications qui suivent.

## Revendications

1. Dispositif pour la fabrication de couches-culottes à ceinture élastique utilisables dans le domaine de l'hygiène infantile et/ou médicale, comprenant des moyens de défilement sous forme de bandes continues de la matière formant la feuille extérieure (18), de la matière formant la ceinture élastique (14), de la matière formant le voile interne (13) et de la matière formant la couche absorbante (17); des moyens de découpe (3) de ladite matière formant la ceinture élastique (14), de façon à former des pavés (15) de ladite matière formant la ceinture élastique (14); des moyens d'encollage (10, 19) de la matière formant le voile interne (13) et de la matière formant la feuille extérieure (18) et des moyens de transfert dudit pavé (15) de matière formant la ceinture élastique (14) collé sur ladite matière formant le voile interne (13) sur ladite matière formant la feuille extérieure (18), caractérisé en ce qu'il comprend des moyens de maintien de la matière formant la bande élastique (14), lesdits moyens agissant depuis le début de l'étirement de ladite matière formant la bande élastiques (14) et pratiquement jusqu'au collage de la matière formant la feuille extérieure (18).

2. Dispositif selon la revendication 1, caractérisé en ce qu'il comprend des premiers moyens d'étirement et de maintien à l'état étiré desdits pavés (15) de matière formant la ceinture élastique (14) avant et pendant le collage de celle-ci sur la matière formant le voile interne (13) et des seconds moyens de maintien desdits pavés (15) de matière formant la ceinture élastique (14) collés sur ladite matière formant le voile interne (13) pratiquement jusqu'au collage de ladite matière formant le voile interne (13) sur ladite matière formant la feuille extérieure (18).

3. Dispositif selon la revendication 1 ou 2, caractérisé en ce que lesdits premiers moyens d'étirement et de maintien comprennent au moins deux poulies d'écartement (6), lesdites poulies (6) comportant chacune une gorge interne et une gorge externe aménagées sur leur surface extérieure, lesdites gorges externes recevant chacune une courroie torique supérieure (11) passant par lesdites gorges externes et par une roue supérieure (5).

4. Dispositif selon l'une quelconque des revendications 1 à 3, caractérisé en ce que les seconds moyens de maintien comprennent au moins deux courroies toriques inférieures (12) passant par les gorges internes desdites poulies d'écartement (6) et sur une partie de la circonférence d'une roue inférieure (7).

5. Dispositif selon l'une quelconque des revendications 1 à 4, caractérisé en ce que les roues supérieure (5) et inférieure (6) sont des roues à vide.

6. Dispositif selon l'une quelconque des revendications 1 à 5, caractérisé en ce qu'il comporte des moyens d'application d'une force de plaquage desdits pavés (15) de matière formant ceinture élastique (14) immédiatement après qu'ils soient collés sur la matière formant le voile interne (13).

7. Dispositif selon la revendication 6, caractérisé en ce que les moyens d'application d'une force de plaquage comprennent des rouleaux (8a) venant en appui, au moins temporairement, contre le rouleau (7) recevant les pièces venant d'être assemblées par collage.

8. Dispositif selon l'une quelconque des revendications 1 à 7, caractérisé en ce qu'il comporte des moyens d'application d'une force de plaquage de la matière formant la feuille extérieure (18) après collage de la matière formant le voile interne (13) munie desdits pavés (15) de matière formant la ceinture élastique (14).

9. Dispositif selon la revendication 8, caractérisé en ce que le moyen d'application d'une force de plaquage comprend un ou plusieurs rouleaux (20) tournant en appui contre la roue inférieure (7), lesdits rouleaux (20) étant maintenus contre ladite roue inférieure (7) par un ou plusieurs moyens élastiques.

10. Dispositif selon la revendication 9, caractérisé en ce que les moyens élastiques consistent en des ressorts.

11. Couche-culotte à ceinture élastique utilisable pour l'hygiène infantile et/ou médicale, formée d'une partie avant et d'une partie arrière réunies par une partie centrale formant l'entrejambe, ladite couche comprenant :
une feuille extérieure imperméable (18);
un voile interne perméable de confort (13);
une couche absorbante (17) disposée entre ladite feuille extérieure (18) et ledit voile interne (13), ladite couche absorbante (17) étant éventuellement munie d'un tampon central (16);
ladite couche-culotte présentant en outre des moyens d'élastification (15) de la ceinture ainsi que des moyens de fermeture (21) de la couche au niveau de la ceinture, caractérisée en ce que lesdits moyens d'élastification (15) comprennent une bande élastique (14) d'une épaisseur comprise entre environ 50 µm et environ 90 µm, de préférence entre environ 50 µm et environ 70 µm.

12. Couche-culotte selon la revendication 11, caractérisée en ce que la dite bande élastique (14) est réalisée à partir de matière plastique, notamment à partir de polypropylène, à partir de latex, à partir de caoutchouc naturel ou synthétique, ainsi que les dérivés et/ou mélanges de ces produits.

13. Couche-culotte selon la revendication 11 ou 12, susceptible d'être obtenue par le dispositif selon l'une quelconque des revendications 1 à 10.

14. Couche-culotte selon l'une quelconque des revendications 11 à 13, caractérisée en ce que ladite bande élastique possède un étirement maximal par rapport à sa limite d'élasticité d'environ 80 %, par rapport à sa longueur initiale.
